# EUROPEAN PATENT SPECIFICATION

(11) **EP 3 710 594 B1**
(45) Date of publication and mention of the grant of the patent: **19.07.2023**
(21) Application number: 18879169.3
(22) Date of filing: 16.11.2018
(51) Int. Cl.: C12Q 1/6806, C12Q 1/25

(54) **METHODS TO MEASURE FUNCTIONAL HETEROGENEITY AMONG SINGLE CELLS**
VERFAHREN ZUR MESSUNG DER FUNKTIONELLEN HETEROGENITÄT ZWISCHEN EINZELNEN ZELLEN
PROCÉDÉS DE MESURE DE L'HÉTÉROGÉNÉITÉ FONCTIONNELLE ENTRE DES CELLULES INDIVIDUELLES

(30) Priority: 16.11.2017 US 201762587320 P
(43) Date of publication of application: 23.09.2020
(73) Proprietor: The Regents of The University of Colorado, A Body Corporate, Denver, CO 80203 (US)
(72) Inventor: HESSELBERTH, Jay, Denver Colorado 80207 (US); RICHER, Amanda, L., Denver Colorado 80207 (US)
(74) Representative: TLIP Limited
(86) International application number: PCT/US2018/061627
(87) International publication number: WO 2019/099906

(56) References cited:
- WO-A1-2017/031378
- WO-A1-2017/053905
- US-A1- 2007 009 944
- US-A1- 2007 166 810
- US-A1- 2010 286 290
- US-A1- 2010 291 564
- US-A1- 2013 115 598
- US-A1- 2015 018 228
- US-A1- 2016 053 253
- Richer Amanda L ET AL: "Simultaneous measurement of biochemical phenotypes and gene expression in single cells", Nucleic acids research, 4 June 2020 (2020-06-04), pages e59-e59, XP055815186, England DOI: 10.1093/nar/gkaa240 Retrieved from the Internet: URL:https://www.researchgate.net/publicati on/340655721_Simultaneous_measurement_of_b iochemical_phenotypes_and_gene_expression_ in_single_cells/fulltext/5eda74fb299bf1c67 d41f64c/Simultaneous-measurement-of-bioche mical-phenotypes-and-gene-expression-in-si ngle-cells.pdf [retrieved on 2021-06-17]

## Description

### CROSS REFERENCE TO RELATD APPLICATION

This application claims the benefit of U.S. Provisional Patent Application Serial No. 62/587,320, filed November 16, 2017,.

### TECHNICAL FIELD

This disclosure relates to methods and systems for single-cell DNA repair capacity evaluation within microfluidic droplets for research and diagnostic identification.

### BACKGROUND

DNA is repaired by multiple different and often redundant pathways including base excision repair (BER), nucleotide excision repair (NER), mismatch repair (MMR) and direct reversal. The importance of DNA repair pathways in cancer is underscored by germline mutations in DNA repair factors that cause cancer predisposition syndromes and the induction of DNA repair activities in response to genotoxic chemotherapy, which represents a major form of drug resistance.

DNA repair capacity varies significantly from person to person and thus the same DNA lesions can have very different mutagenic outcomes depending on genetic background and physiological state. Moreover, this heterogeneity partially accounts for differences in how patients respond to chemotherapeutic treatments. Interest in targeting DNA repair pathways for therapeutic benefit has grown and many new therapies inhibit specific DNA repair factors.

Current methods used to measure DNA repair capacity have drawbacks. For example, DNA repair capacity can be estimated from mRNA and protein levels, but these correlations are not always predictive of repair levels. Additionally, for practical reasons, the techniques that measure mRNA or protein levels are usually conducted on samples comprising thousands to millions of cells, but this has hindered direct assessment of individual cells - the fundamental unit of biology.

The comet assay provides a crude measure of DNA damage in single cells but can be modified to study specific types of damage. Microarrays and single probes programmed with specific DNA repair substrates have been used to study DNA repair, but this approach provides a bulk measure of DNA repair in cells or extracts with no insight in cell-to-cell variation. The oligonucleotide probe recovery assay enables study of a recently developed host cell reactivation (HCR) assay and measures the repair of specific adducts on defined DNA templates. This assay has been used to study the impact of chemotherapeutic treatments on multiple DNA repair pathways. But a drawback of the HCR assay is that it requires the production and transfection of reporter plasmids, limiting its utility for studying primary cells. It also measures complete repair of adducts without capturing many informative steps of the repair process, and is restricted to a small number of substrates interrogated in a single experiment (maximum of 4 at a time).

US2010/286290 relates to an enzyme activity assay using rolling circle amplification.

Thus, new tools that incisively measure the cellular enzymatic activity, and therefore the functional heterogeneity between cells, and a measure of response to these therapies will be valuable to study variation in enzymatic activity and capacity.

### SUMMARY

The present invention is defined by the appended claims.

The inventor has developed a multiplexed assay for evaluating enzymatic activity (for example DNA repair capacity, protease activity, or kinase activity) that employs highly parallel measurement of enzyme activity on enzyme substrates linked to barcoded oligonucleotides. For example, the DNA repair enzyme substrates may be located on barcoded DNA hairpins (and is therefore referred to as "Haircut"). The method simultaneously measures enzyme action on diverse enzyme substrates with single molecule and nucleotide resolution. Moreover, the method is designed to be compatible with single cell microfluidic analyzers and can be used to study heterogeneity in enzyme activity among thousands of single cells in complex mixtures. The method provides the ability to study multiple enzyme activities in mammalian cell extracts with high specificity and signal-to-noise. This method addresses many of the shortcomings of current methods used to measure enzyme activity and capacity. For example, this method may simultaneously measure the initial steps of DNA repair on many substrates and captures repair products with single-nucleotide precision, providing richer information than what is available from existing assays. Whereas the HCR assay requires transfection of plasmid reporters limiting its application to cells that can be cultured, the methods of this disclosure can be applied to cells without further manipulation, enabling rapid analysis of cultured and primary cells.

The methods of this disclosure may be rapidly expanded to measure many different types of DNA repair events, by measuring DNA repair events on many DNA repair substrates in a single reaction, including those for base excision, mismatch, and nucleotide excision and incision, ribonucleotide excision, topoisomerase-mediated ribonucleotide repair, homologous recombination (HR), non-homologous end joining (NHEJ), translesion DNA synthesis (TLS), and direct reversal. Additionally, small molecule inhibitors may be used to determine how the loss of specific repair activities affects the recovery of DNA repair events. In these methods, the cellular extracts may be supplemented with recombinant repair enzymes to probe the biochemical requirements for DNA repair in the assay. Variations in DNA repair capacity may be assessed between different cell types, providing a basis for understanding how genetic or pharmacologic perturbations change DNA repair capacity. These methods may also be used to determine how cancer chemotherapies change the capacity of cells to repair DNA, enabling the use of these methods as a prognostic and diagnostic tool for cancer therapy.

These methods may be used to measure DNA repair capacity in single cells. Heterogeneity in DNA repair capacity contributes to clonal resistance and expansion during chemotherapeutic treatment, but the contribution of heterogeneity has not been widely explored in single cells among complex populations. The methods of this disclosure may be miniaturized to a single cell by capturing or encapsulating single cells and beads in a nanowell, or in a water-in-oil emulsion using droplet microfluidics, or within microfluidic channels between pressure-controlled valves. Upon capture or encapsulation, a cell is lysed to release repair activities into the reaction chamber, which act on the DNA substrates utilized in the methods of this disclosure, which may be bead-immobilized DNA substrates. The repair products are recovered and analyzed, and repair activities are assigned to single cells via barcode sequences that are unique to the substrates on each bead. The inventor's data demonstrate that DNA repair activity may be detected at concentrations expected upon lysis of a single cell in a 50 picoliter reaction. Implemented in conjunction with a microfluidic analyzer, repair capacity may be measured in 100,000 cells in a single experiment. These methods are therefore capable of measuring heterogeneity in multiple DNA repair pathways simultaneously in single cells present in complex mixtures (for example during normal growth or during cancer treatment), measuring heterogeneity in DNA repair capacity for several immortalized and primary cell types under normal growth conditions, and determining how genotoxic chemotherapies may alter heterogeneity in simple and complex cell populations.

This Summary is neither intended nor should it be construed as being representative of the full extent and scope of the present invention. Moreover, references made herein to "the present disclosure," or aspects thereof, should be understood to mean certain embodiments of the present disclosure and should not necessarily be construed as limiting all embodiments to a particular description. The present disclosure is set forth in various levels of detail in this Summary as well as in the attached figures and the Detailed Description and no limitation as to the scope of the present disclosure is intended by either the inclusion or non-inclusion of elements, components, etc. in this Summary. Additional aspects of the present invention will become more readily apparent from the Detailed Description, particularly when taken together with the figures.

### BRIEF DESCRIPTION OF FIGURES

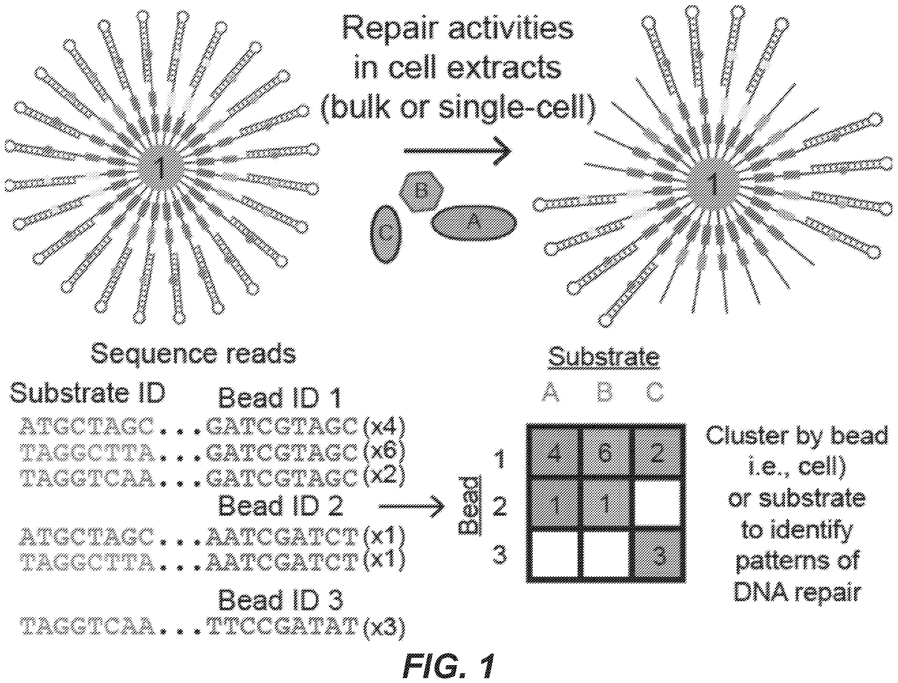

**FIG. 1** provides an overview of the DNA repair assessment methods ("Haircut") of this disclosure. Bead-immobilized products of DNA repair are recovered and sequenced to quantify repair capacity in bulk extract or single cells. Three hypothetical substrate IDs are depicted: ATGCTAGC (SEQ ID NO:1); TAGGCTTA (SEQ ID NO:2); TAGGTCAA (SEQ ID NO:3). Three hypothetical bead IDs are depicted: GATCGTAGC (SEQ ID NO:4); AATCGATCT (SEQ ID NO:5); TTCCGATAT (SEQ ID NO:6).

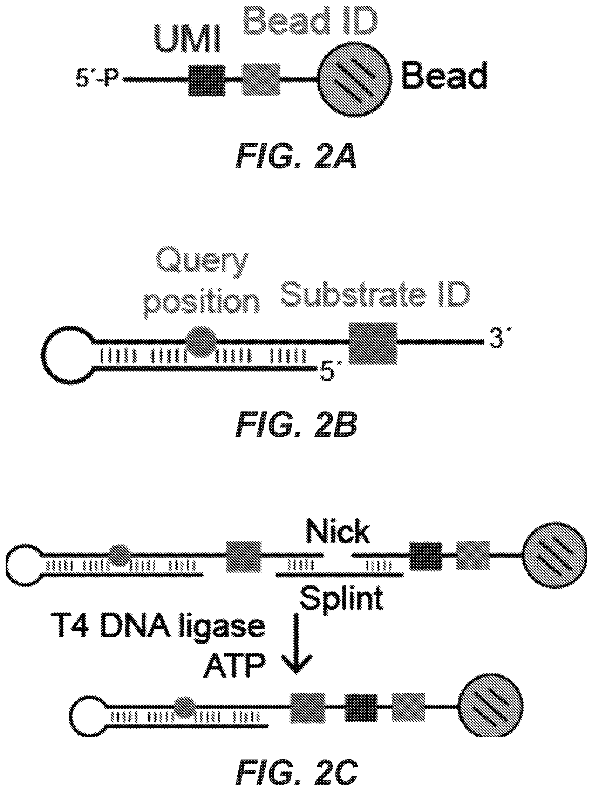

**FIGS. 2A-2C** depict test oligonucleotide synthesis on beads. **FIG. 2A** depicts individual oligonucleotides synthesized on beads containing a bead ID (each oligo on a bead has the same bead ID) and a unique molecular index (UMI; each oligo on a bead has a UMI). **FIG. 2B** shows hairpin repair substrates (approx. 100 nt in size) are synthesized as separate oligonucleotides with unique (approx. 15 bp) sequences ("Substrate IDs") for each repair substrate. **FIG. 2C** depicts how each repair substrate is covalently attached to beads by splinted ligation.

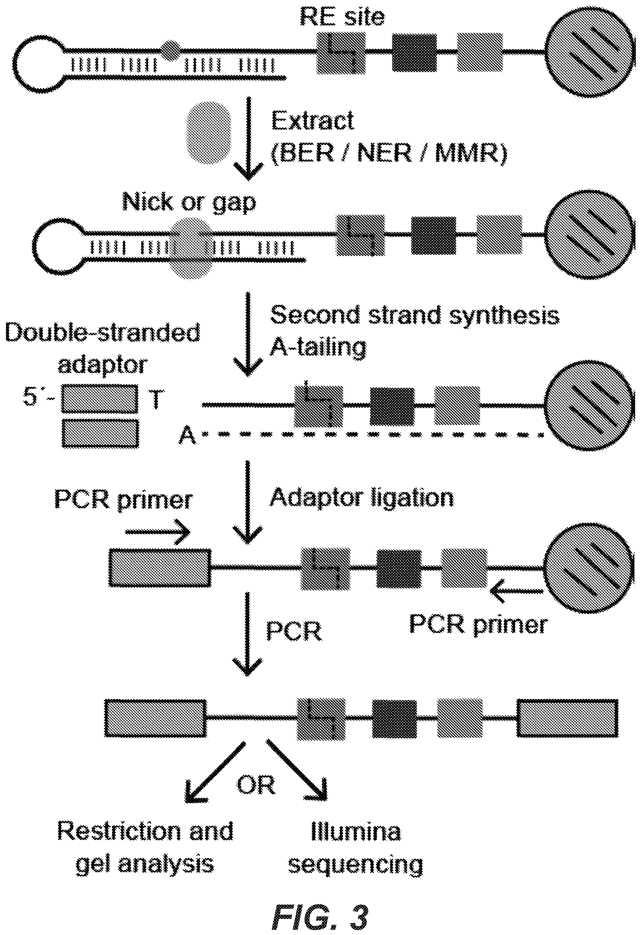

**FIG. 3** depicts beads incubated with cellular extract wherein DNA repair enzymes in the cellular extract create nicks or gaps in the repair substrate. After incubation, repaired products are recovered by second strand synthesis, ligation of double stranded adaptors, and PCR amplification. PCR products are purified and analyzed by restriction analysis or DNA sequencing.

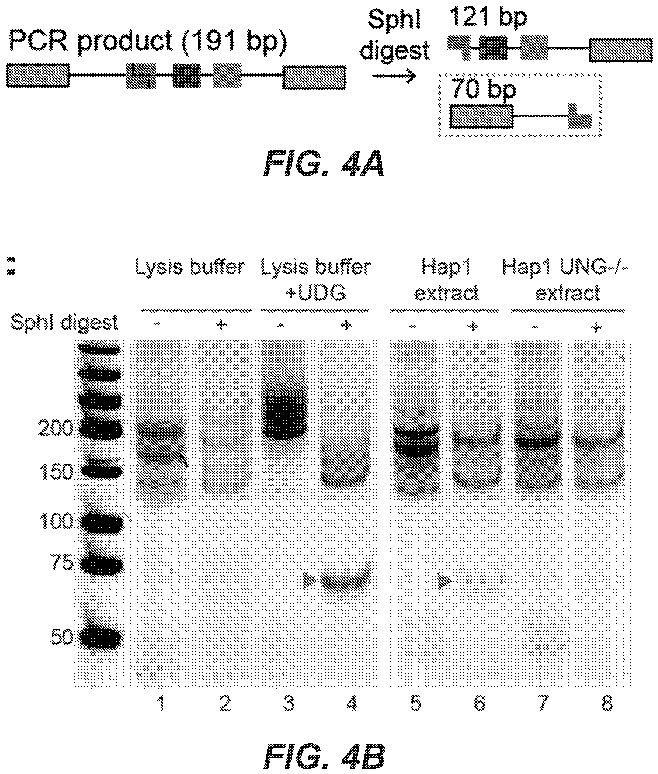

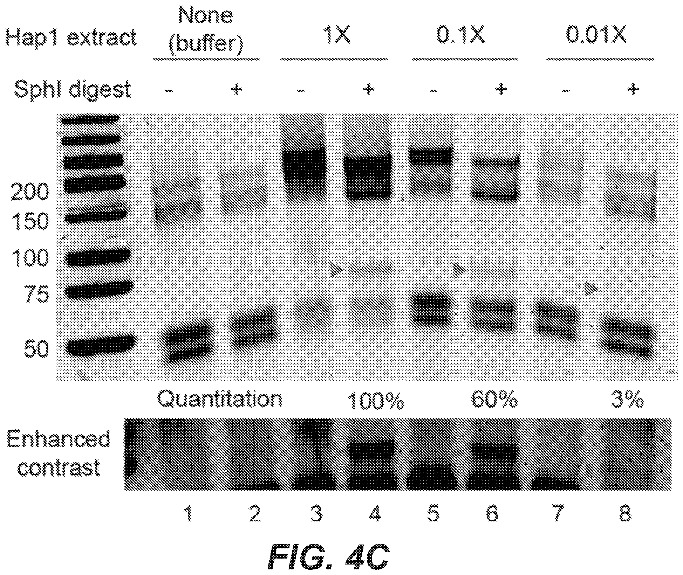

**FIG. 4A** depicts the Sphl digestion of PCR products that were recovered from excision of an A-U repair substrate to yield 121 bp and 70 bp products (**FIG. 4B****,** arrowheads). **FIG. 4B** shows the repair of the A-U hairpin repair substrate depicted in **FIG. 4A****,** using lysis buffer with and without recombinant UDG, as well as extracts from Hap1 and UNG-/- derivative cells. **FIG. 4C** shows products of repair detected at 100-fold below the concentration (1X) expected upon single cell lysis in a 50 pL droplet.

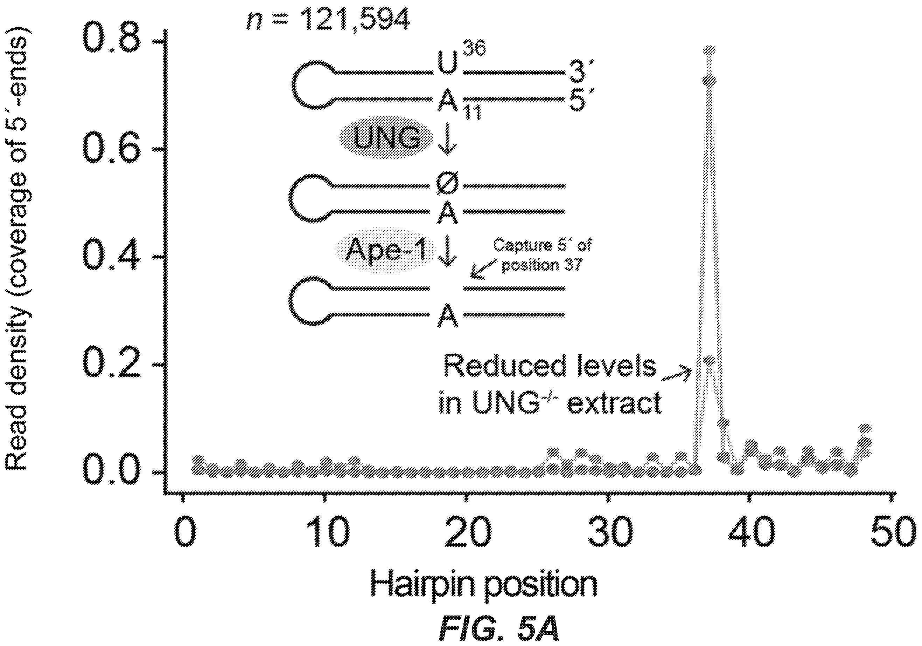

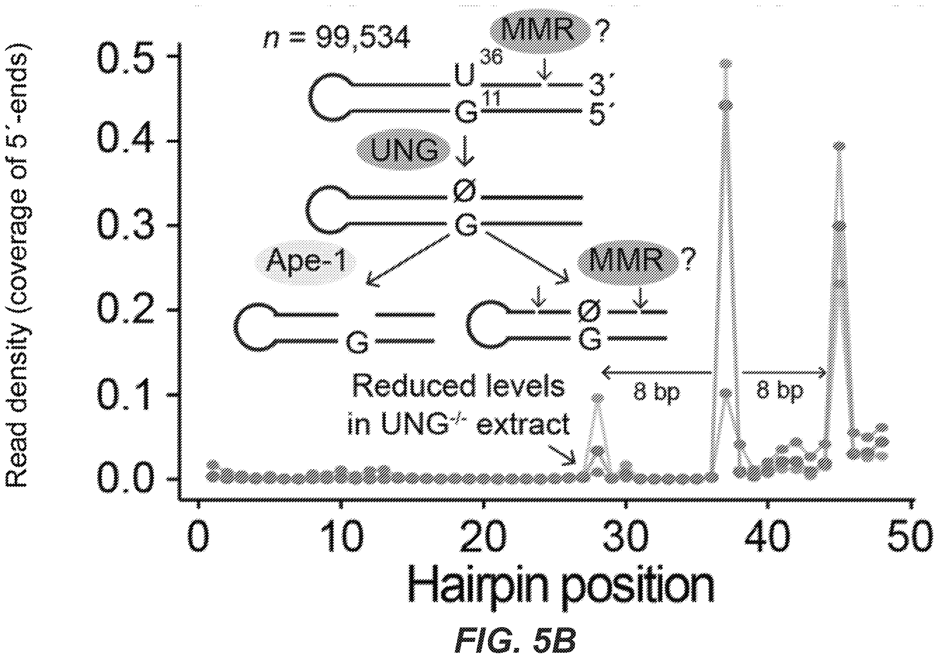

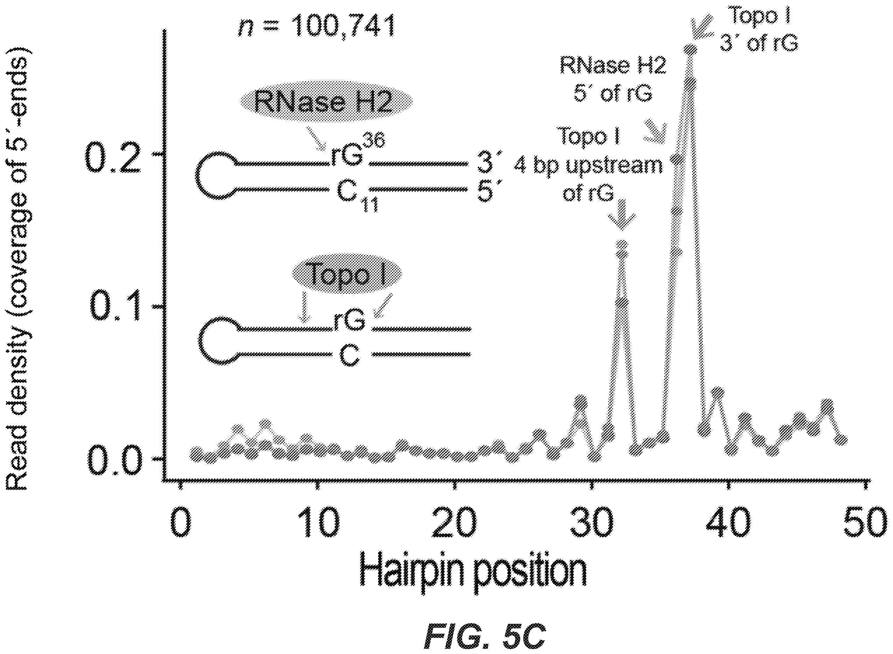

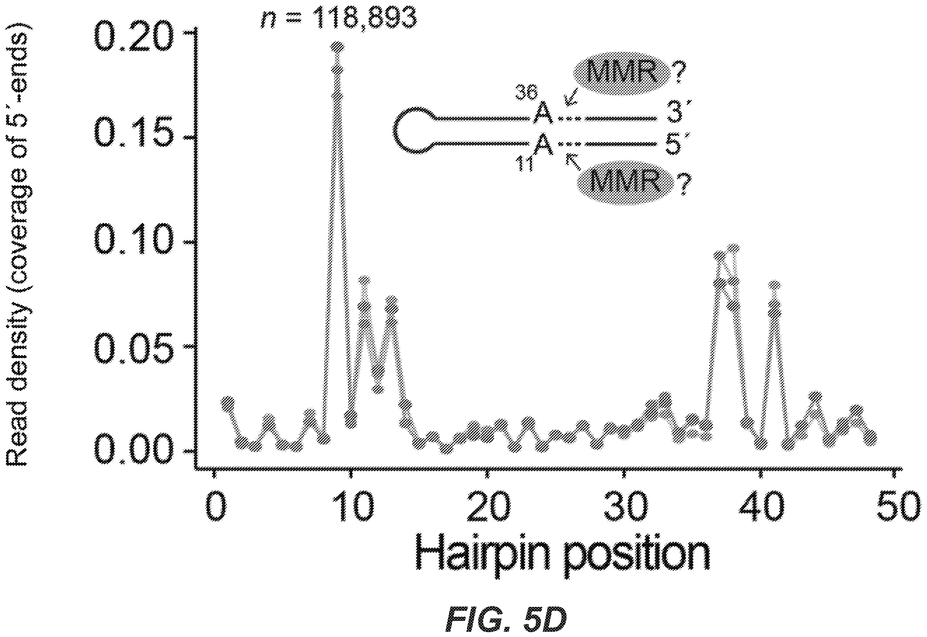

**FIGS. 5A-5D** show the analysis of hairpin substrate repair by DNA sequencing. A mixture of hairpin substrates was treated with extracts from different cell lines and analyzed by Illumina sequencing (method depicted in **FIG. 3**). Coverage of 5'-termini was calculated for repair products and plotted versus hairpin position. The total reads are listed for Hap1 cells for each panel (top left); reads of similar magnitude were obtained for all cell extracts. Hairpins are numbered from 5' to 3' with query positions at 36 (base-paired to position 11). **FIG. 5A** shows that the repair of the A-U hairpin repair substrate is catalyzed by sequential action of UNG to remove the uracil nucleobase and Ape1 to remove the abasic site. The large signal at position 37 is one base downstream of the uracil and is reduced in UNG-/- cells by about 75%. **FIG. 5B** shows that the repair of the G-U hairpin is a combination of UNG-mediated uracil excision (position 37), and symmetric cleavage of 8 nt up- and downstream of the query position, possibly by mismatch repair (MMR) factors. In UNG-/- cells, uracil excision is reduced (position 37, blue) and only the downstream incision is observed, suggesting that an abasic site is required to generate the upstream incision. **FIG. 5C** shows that the repair of the rG-C (with a single ribonucleotide) hairpin is a combination of incision by RNase H2 (position 36), and two independent cleavages likely catalyzed by Topoisomerase I (positions 32 and 37). **FIG. 5D** shows that the repair of a mismatched A-A hairpin is likely catalyzed by mismatch repair (MMR) factors with most cleavage localized near the mismatch (highest signals at positions 9 and 37/38).

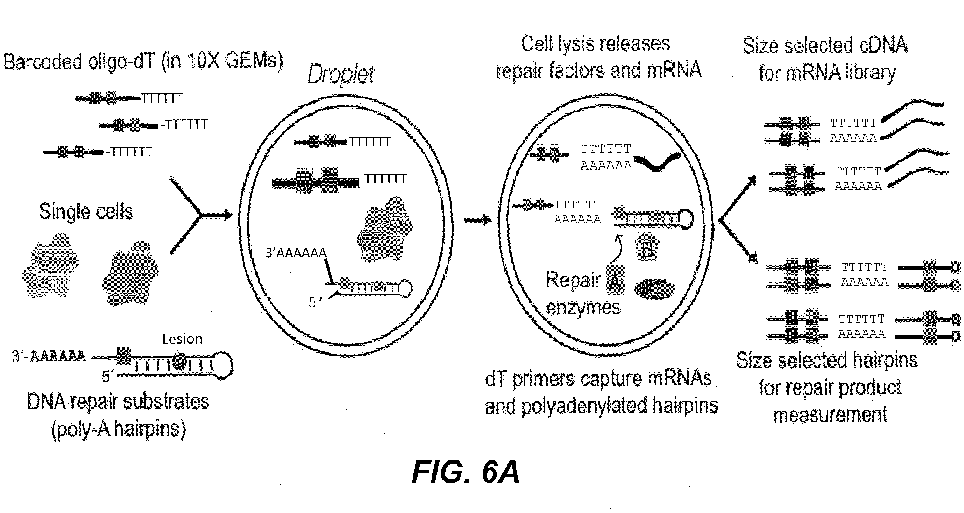

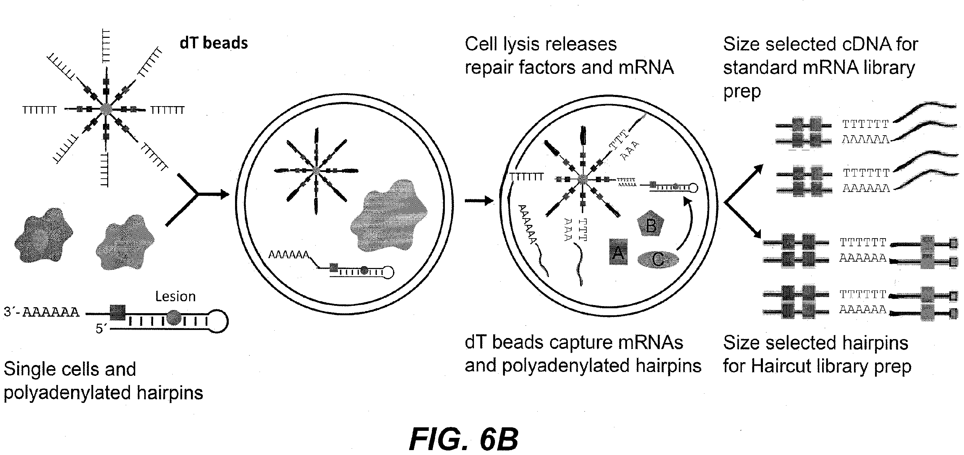

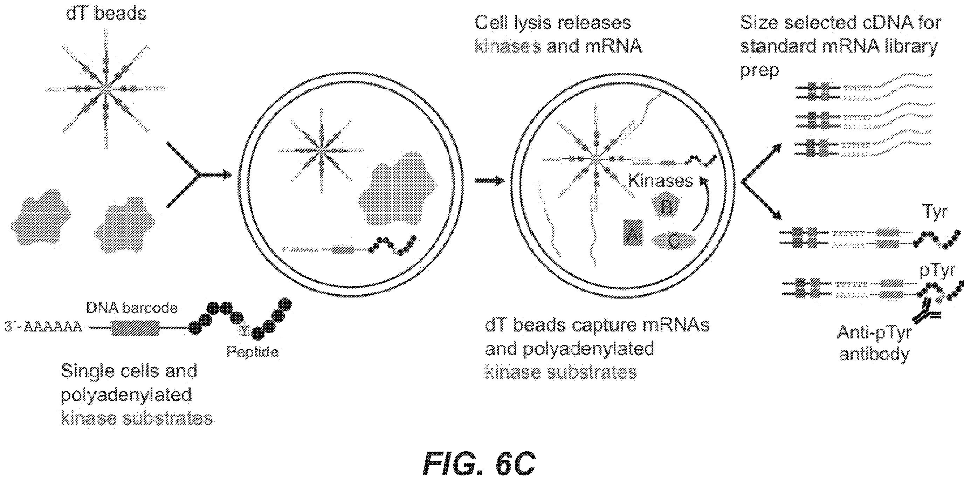

**FIG. 6A** is a schematic depiction of a method of measuring DNA repair activities in single-cell extracts using polyadenylated DNA hairpin substrates with defined chemical modifications to measure the activity of cognate DNA repair enzymes by analyzing their conversion to repair intermediates and products. In these assays, the poly-A tails (AAAAAA; SEQ ID NO:7) on the DNA repair substrates bind with the poly-T tails (TTTTTT; SEQ ID NO:8) on the molecular tags. **FIG. 6B** is a schematic depiction of a similar method of measuring DNA repair activities in single-cell extracts using polyadenylated DNA hairpin substrates with defined chemical modifications to measure the activity of cognate DNA repair enzymes by analyzing their conversion to repair intermediates and products, adapted for use with a solid support (polymeric beads). **FIG. 6C** is a schematic depiction of a method of measuring kinase activities in single-cell extracts using a peptide enzyme substrate including a tyrosine phosphorylation site linked to the DNA barcode to measure the activity of kinase enzymes by analyzing the phosphorylation of the tyrosine site using an anti-pTyr antibody. In these figures, the poly-A tails (AAAAAA; SEQ ID NO:7) on the DNA repair substrates bind with the poly-T tails (TTTTTT; SEQ ID NO:8) on the molecular tags.

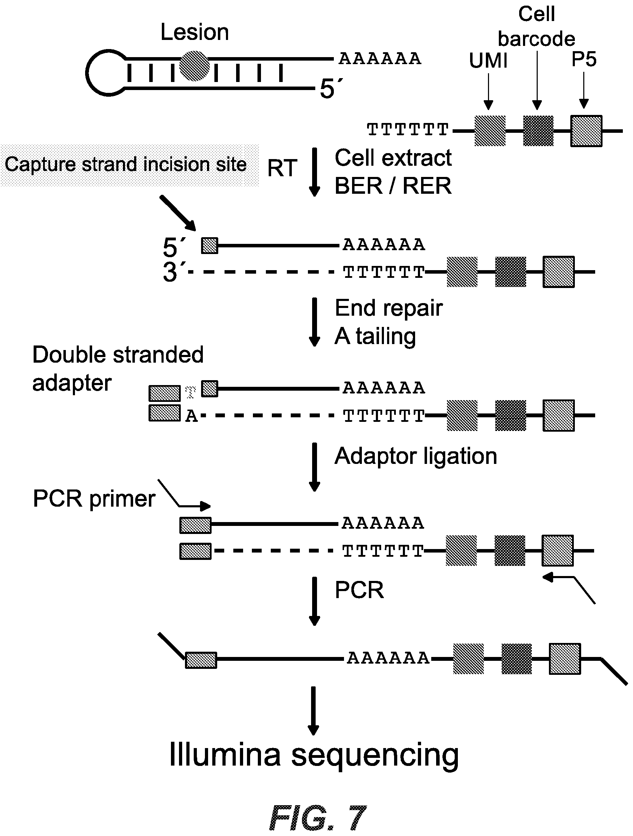

**FIG. 7** shows a flow diagram of the steps in an analytical method of this disclosure in which the enzyme substrates remain in soluble form (i.e., in the absence of a solid support that captures the enzyme substrates). The illustrated method leads to the identification of the products of DNA repair events (RER Ribonucleotide Excision Repair; BER Base Excision Repair) by high-throughput DNA sequencing (including, for example, Illumina sequencing) to count repair events in each single-cell reaction. The poly-A tails (AAAAAA; SEQ ID NO:7) on the DNA repair substrates bind with the poly-T tails (TTTTTT; SEQ ID NO:8) on the molecular tags.

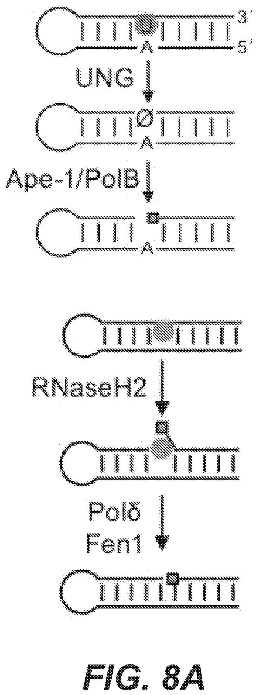

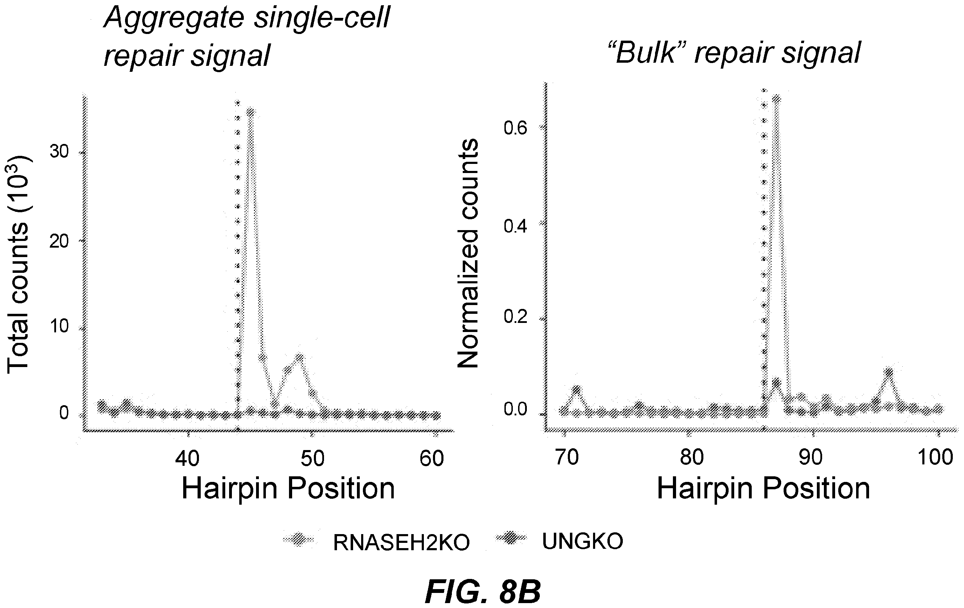

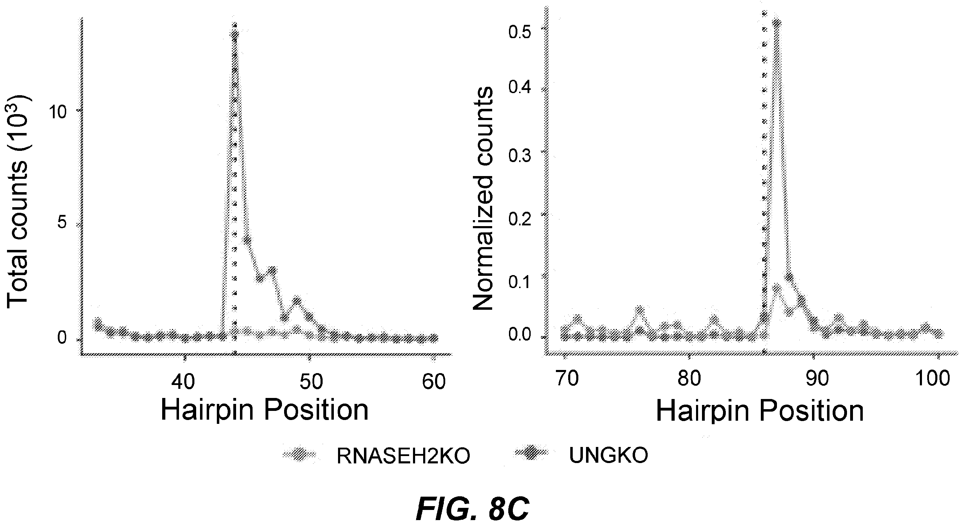

**FIG. 8A** depicts expected sites of DNA repair reactions in a single-cell mRNA sequencing experiment conducted with a mixture of two Hap1 cell lines with deletions in either the UNG gene (catalyzes uracil excision) or RNASEH2C (catalyzes ribonucleotide excision). Polyadenylated hairpins containing either a uracil or a ribonucleotide were added to the single cell suspension prior to capture. Expected sites of incision are marked with grey boxes. FIG. 8B. shows the count of cells scored as either UNGKO **(****FIG. 8B****)** or RNASEH2CKO **(****FIG. 8C****).**

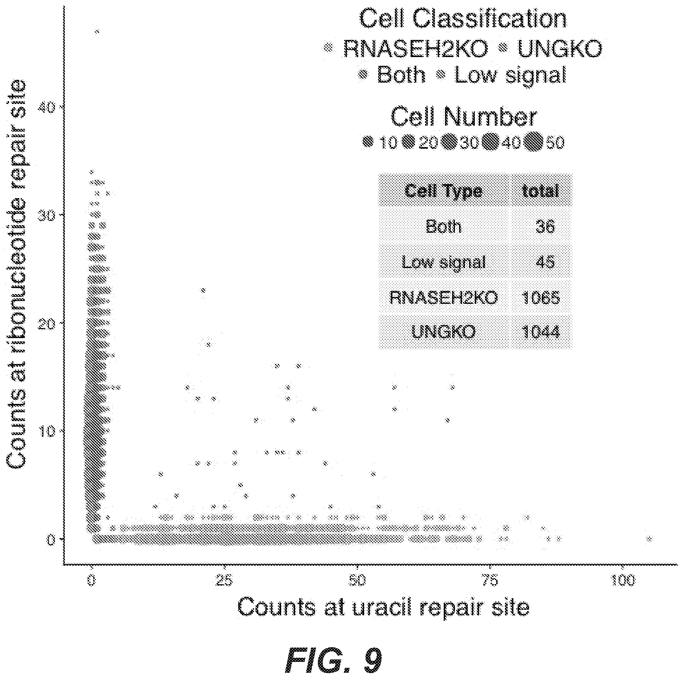

**FIG. 9** shows the results of an analysis of cell mixing was evaluated using a "functional barnyard" approach using a mixture of UNGKO and RNASEH2CKO Hap1 cells. DNA repair activities are plotted at different levels of repair for each detected cell with dots scaled to the number of cells in each bin.

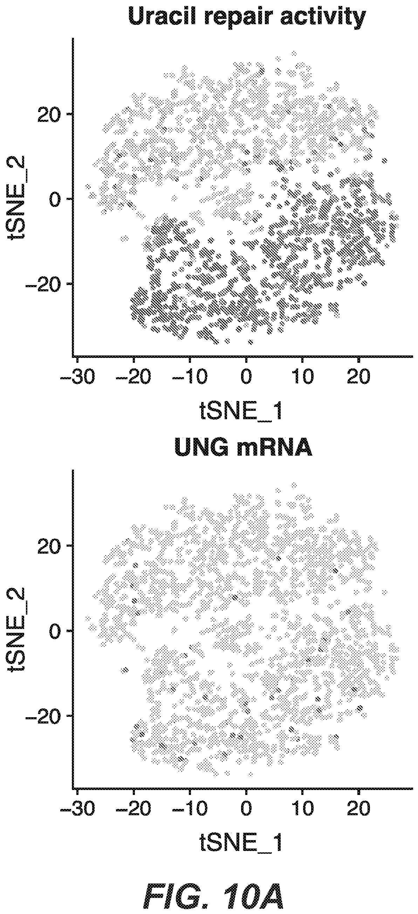

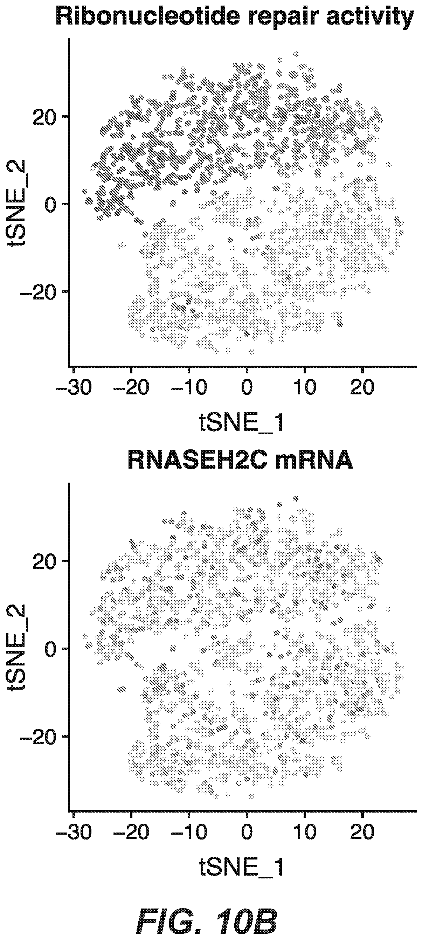

**FIGS. 10A** **and** **10B** show t-SNE projection plots of cell types clustered by mRNA expression to identify major classes. Uracil repair activity (**FIG. 10A**; top) and ribonucleotide repair activity (**FIG. 10B**; top) were superimposed and clearly delineate the two major cell types in the experiment. For comparison Levels of UNG and RNASEH2C mRNA are plotted on the bottom panels; these data alone are not sufficient to classify cell types.

### DETAILED DESCRIPTION

This disclosure provides methods of analyzing multiple enzymatic activities in single cells in parallel in a sequencing-based assay. For example, these methods may be used to characterize DNA repair activities in individual mammalian cells. The methods are essentially a massively parallel measurement of enzymatic activity (such as DNA strand incision by DNA repair enzymes). Because strand incision initiates most repair events and most repair activities have been shown to function in vitro on defined substrates, a broad range of DNA repair activities can be captured in certain embodiments of these assay methods.

The methods of this disclosure, as conducted on a single cell comprise the formation of a reaction containing a single mammalian cell and one or more oligonucleotides linked to an enzyme substrate. In these methods, the enzyme substrate may be in the form of a hairpin double stranded DNA sequence, or an oligonucleotide-linked peptide comprising a specific enzyme recognition sequence. in these methods, the oligonucleotide-linked enzyme substrate may be linked to a solid support or remain soluble in the assay compositions until the final analysis of enzyme activity in the cell. The reaction may be formed in a droplet of an emulsion, or within a subnanoliter well (a "nanowell" comprising a nanoliter-sized well) which may be formed as a dense array of nanowells to examine individual cells or monolayers of cells, or within microfluidic channels between pressure-controlled valves (see, for example, Prakadan, SM., Nature Reviews Genetics, 2017 Jun;18(6):345-361).

The one or more oligonucleotides include an enzyme substrate, such as a peptide recognition sequence for a protease, or a phosphorylation site for kinases, or a DNA adduct that will act as a substrate for DNA repair activities, performed by the cellular contents of the single mammalian cell. The one or more oligonucleotides also include i) a cell identification sequence associated with a specific cell (i.e., a "cell barcode"), ii) a substrate identification sequence that is associated with a specific type of enzyme substrate (i.e., a "DNA barcode"), and, iii) a unique molecular identifier sequence.

The term "barcode", as used herein, generally refers to a label that may be attached to a polynucleotide, or any variant thereof, to convey information about the polynucleotide. For example, a barcode may be a polynucleotide sequence attached to all fragments of a target polynucleotide contained within a particular partition. This barcode may then be sequenced with the fragments of the target polynucleotide. The presence of the same barcode on multiple sequences may provide information about the origin of the sequence. For example, a barcode may indicate that the sequence came from a particular cell. This may be particularly useful when several partitions are pooled before sequencing.

In these methods, the one or more oligonucleotides may be linked to a solid substrate, such as a bead, to facilitate capture and washing of the one or more oligonucleotides from other cellular components prior to analysis of the enzymatic activity. Alternatively or additionally, the one or more oligonucleotides may remain soluble throughout the assay steps.

This, in an exemplary embodiment, the one or more oligonucleotides may include i) an individual bead identification sequence, ii) a substrate identification sequence that is associated with a specific type of enzyme substrate, and, iii) a unique molecular identifier sequence.

The single mammalian cell in these reactions is lysed to expose the one or more oligonucleotides to the cellular contents of the lysed cell. These reactions, which contain the cellular contents from the lysed cells and the one or more oligonucleotides are incubated under conditions in which the cellular contents may act on the enzyme substrates. For example, DNA repair enzymes in the cell lysate may repair the DNA adduct present in the one or more oligonucleotides, or kinases present in the cell lysate may phosphorylate or dephosphorylate the enzyme substrate or proteases present in the cell lysate may cleave the enzyme substrate. After sufficient incubation, the one or more oligonucleotides (or the solid support linked to the one or more oligonucleotides) is separated from the reaction components.

The oligonucleotides linked to the solid support may be reacted with an abasic endonuclease to repair apurinic/apyrimidinic (AP) sites in the oligonucleotide by catalyzing hydrolytic incision of the phosphodiester backbone of the oligonucleotide immediately adjacent to the adduct, followed by phosphorylation of the 5' end of the oligonucleotide to generate 5' ends that are competent for ligation.

A single strand DNA adaptor is ligated to the phosphorylated 5' end of the oligonucleotide, and the oligonucleotide is amplified by polymerase chain reaction (PCR) to produce amplified DNA products. The PCR products are then analyzed to determine the amount and/or type of enzymatic activity that was applied to the enzyme substrate in the oligonucleotide by the cellular contents from the lysed cell(s). This analysis may include RFLP analysis that indicates whether a type of enzymatic action took place. This analysis may include DNA sequencing to determine the identity of the bead identification sequence, and the unique molecular identifier sequence to determine the types of enzymatic action that were applied to the enzyme substrates by each cell. Thus, the enzymatic activity or capacity of an individual cell can be ascertained and evaluated by these methods.

In these methods, the mammalian cell may be derived from, but is not limited to, cells excised from a living tissue (such as a biopsy sample), a cell from a population of cultured cells, cells shed from a tissue, a cell in the blood circulation of a mammal, cells washed and recovered during a surgery, a cell that has been treated with an agent (such as a chemotherapeutic agent), a tumor cell, an immunological cell (i.e., a cell that forms part of the immune system of an organism), a cell that has been obtained or isolated from a body fluid source from a mammalian body fluid such as blood, urine, sweat, sputum, feces, cerebrospinal fluid, ascites, pleural effusion, bile, pancreatic fluid, and the like. Preferably, the cell is a human cell.

Methods of lysing cells are well-known in the art and within the methods of this disclosure may include various means of freeze-thaw disruption, osmotic disruption, mechanical disruption, ultrasonic disruption, enzymatic disruption (e.g., hyaluronidase, dispase, proteases, and nucleases (for example, deoxyribonuclease and ribonuclease)), or chemical disruption (non-ionic detergents such as, alkylaryl polyether alcohol (TRITON^{™} X-100), octylphenoxy polyethoxyethanol, BRIJ-35, a polyethoxyethanol lauryl ether, polysorbate 20 (TWEEN 20^{™}), a polyethoxyethanol sorbitan monolaureate, polyethylene lauryl ether, and ionic detergents, such as sodium dodecyl sulphate, sulfated higher aliphatic alcohols, sulfonated alkanes and sulfonated alkylarenes, or combinations thereof.

The lysing of the single mammalian cell and the incubation of the cellular contents with the oligonucleotides is preferably conducted in a small volume reaction chamber (e.g., microfluidic channel, emulsion droplet, nanowell) containing the single cell and one or more oligonucleotides linked to a solid support. These small volume reaction chambers may contain a uniform population of oligonucleotides or a variety of different oligonucleotides, each containing an enzyme substrate that may be acted upon by enzymes present in the cellular contents of the lysed single mammalian cell. within these reaction chambers, the oligonucleotides containing the enzyme substrate may be soluble or may be linked one or more solid supports.

In a preferred embodiment of these methods, the small volume reaction chamber is in the form of a droplet within a droplet-based microfluidic device. Microfluidic technology offers a methodology for the rapid generation of monodisperse microdroplets that can be used as miniaturized reactors for high-sensitivity single-cell analysis. Single cells are compartmentalized within the discrete aqueous droplets surrounded by an immiscible carrier oil, which reduces the possibility of cross-contamination among different cells. Due to the controllable droplet size and uniformity, the droplet content (e.g. the reagent composition and concentration) can also be precisely tuned to provide the desired microenvironment for individual cell reactions. The ultralow volume (femtoliter to nanoliter) of the reactions means that the enzymes and other biomolecules from a single cell are highly concentrated and detectable. Furthermore, the droplet technology allows massively parallel handling of millions of independent reactions with high throughput, thereby enabling the analysis of vast populations of single cells to detect rare events or to probe cellular heterogeneity (e.g., heterogeneity in DNA repair capacity in populations of cells). Exemplary droplet-based platforms designed for use in single-cell genomics analysis and useful in the analytical methods of this disclosure are commercially available (for example, Chromium^{™} from 10x Genomics, ddSEQ^{™} from Bio-Rad Laboratories, InDrop^{™} from 1CellBio, and µEncapsulator^{™} from Dolomite Bio/Blacktrace Holdings).

In these methods the microfluidic device enables the manipulation of discrete fluidic packets in the form of picoliter droplets (typically 20-80 picoliter volume droplets; preferably 50pL volume droplets) and addresses the need for lower costs, higher throughput, and higher sensitivities at which these DNA repair evaluation methods can be performed. The technique is well adapted to perform operations and manipulations in series, like encapsulation and screening. In particular, microfluidic devices enable the screening of individual cells in individual droplets using fluorescence-based techniques or mass spectrometry, to sort droplets from other droplets, to store them, to re-inject them into other microfluidic devices, to fuse droplets with other droplets and to incubate cells (or the contents of lysed cells) within droplets. Thus, by encapsulating single cells in individual droplets, cellular contents can be easily isolated providing inexpensive small volume reaction vessels for research and diagnostic applications. The act of lysing a single cell within a droplet in an emulsion may include immersing the encapsulated cell droplet in a cell lysis buffer to dissolve the cellular protein and membrane and release the cellular contents into the droplet within the emulsion, which may be within a microfluidic device. The droplets may be part of an aqueous emulsion in a microfluidic device. For example, the droplet may be in a water-in-oil emulsion (W/O emulsion). The droplets may be made with a microfluidic generator, which may generate the droplets in hydrophobic oil. Many microfluidic devices advantageously employ a fluorous oil as a component of the oil in a W/O emulsion because fluorous oil can store dissolved oxygen. A surfactant may be present to aid in establishing and/or maintaining the emulsion.

Another useful approach for forming a small volume reaction chamber to analyze the response of a single cell uses arrays of nano-wells. Nanowells are roofless, miniature containers fabricated from polydimethylsiloxane (PDMS), glass or other related materials. In a similar way to droplet-based devices, cells or cellular components can be loaded at low densities to achieve, at most, a single element per well, but here, loading can be achieved using gravity alone. Subsequently, the wells can be sealed by the addition of a roof (for example, a glass slide) thereby isolating each cell from its neighbors. If desired, this cap can be selectively permeable or functionalized to help profile cellular analytes, such as secreted cytokines or antibodies. A major advantage of nano-wells is their operational simplicity and sample efficiency. The system requires few peripherals, and small starting numbers of cells can be used. Additionally, the fixed spatial locations of each well can be used to link several discrete measurements.

A third useful approach for forming a small volume reaction chamber to analyze the response of a single cell uses microfluidic channels coupled with pressure-controlled valves. Valve-based systems typically rely on a soft elastomeric membrane that can be deflected with pressure to block flow through a microchannel such that the valves seal a channel to confine an individual cell. In these systems, several valves can be combined to actuate a complex series of operations in space and time, such as adding, removing, or mixing reagents. Integrated fluidic circuits, which contain multiple channels and valves, can process many individual cells with limited manual input. However, these systems typically require larger volumes than both droplet- and nanowell-based single-cell analysis technologies.

Any small volume reaction chamber described herein may comprise multiple partitions. For example, a partition may comprise 1, 2, 3, 4, 5, 6, 7, 8, 9, 10, 11, 12, 13, 14, 15, 16, 17, 18, 19, 20, 50, 100, 500, 1000, 1500, 2000, 2500, 3000, 3500, 4000, 4500, 5000, 5500, 6000, 6500, 7000, 7500, 8000, 8500, 9000, 9500, 10000, or 50000 partitions. A partition may comprise at least 1, 2, 3, 4, 5, 6, 7, 8, 9, 10, 11, 12, 13, 14, 15, 16, 17, 18, 19, 20, 50, 100, 500, 1000, 1500, 2000, 2500, 3000, 3500, 4000, 4500, 5000, 5500, 6000, 6500, 7000, 7500, 8000, 8500, 9000, 9500, 10000, or 50000 partitions. In some cases, a partition may comprise less than 3, 4, 5, 6, 7, 8, 9, 10, 11, 12, 13, 14, 15, 16, 17, 18, 19, 20, 50, 100, 500, 1000, 1500, 2000, 2500, 3000, 3500, 4000, 4500, 5000, 5500, 6000, 6500, 7000, 7500, 8000, 8500, 9000, 9500, 10000, or 50000 partitions. In some cases, each partition may comprise 2-50, 2-20, 2-10, or 2-5 partitions.

Such partitions may be pre-loaded with reagents to perform a particular reaction. For example, one or more reagents may be placed within a nanowell. The contents of the nanowell may include, for example, oligonucleotide-linked enzyme substrates, restriction enzymes, ligases, barcodes, and adapters for processing the polynucleotide sample placed in the nanowell. For example, a droplet of an emulsion may be an aqueous droplet in an oil phase. The droplet may comprise, for example, one or more reagents (e.g., restriction enzymes, ligases, polymerases, reagents necessary for nucleic acid amplification (e.g., primers, DNA polymerases, dNTPs, buffers)), a polynucleotide sample, a barcode sequence, and an oligonucleotide-linked enzyme substrate. In some cases, the oligonucleotide-linked enzyme substrates, polynucleotide sample, or any reagent may be associated with a solid surface within a droplet. In some cases, the solid surface is a bead. In some cases, the bead is a gel bead (see e.g., Agresti et al., U.S. Patent Publication No. 2010/0136544). In some cases, the droplet is hardened into a gel bead (e.g., via polymerization).

A species may be contained within a droplet in an emulsion containing, for example, a first phase (e.g., oil or water) forming the droplet and a second (continuous) phase (e.g., water or oil). An emulsion may be a single emulsion, for example, a water-in-oil or an oil-in-water emulsion. An emulsion may be a double emulsion, for example a water-in-oil-in-water or an oil-in-water-in-oil emulsion. Higher-order emulsions are also possible. The emulsion may be held in any suitable container.

In some cases, droplets in an emulsion may comprise other partitions. A droplet in an emulsion may comprise any suitable partition including, for example, another droplet (e.g., a droplet in an emulsion), a capsule, a bead, and the like. Each partition may be present as a single partition or a plurality of partitions, and each partition may comprise the same species or different species.

The oligonucleotides containing enzyme substrates may be linked to a solid support that is exposed to the cellular contents of the lysed cell. The solid support may be in the form of beads, pellets, disks, and rods. Preferably, the solid support is formed as microbeads that are linked to one or more oligonucleotides, including tens, or hundreds, or thousands, or hundreds of thousands of oligonucleotides. The solid support may be composed of materials such as organic polymers (such as a resin formed of polystyrene or polypropylene); semiconductors including quantum dots (semiconductor nanoparticles) formed of a semiconductor material (such as cadmium selenide (CdSe), zinc sulfide (ZnS), cadmium sulfide (CdS), zinc selenide (ZnSe), or zinc oxide (ZnO)); metals (such as gold); polymers (such as a silica); cellulose or a cellulose derivative; an acrylic resin; glass; silica gel; polystyrene; gelatin; polyvinylpyrrolidone; copolymers of vinyl and acrylamide; divinylbenzene-crosslinked polyacrylamide; a latex gel; polystyrene; dextran; rubber; silicon; plastic; nitrocellulose; natural sea sponge, cross linked dextran (e.g., SEPHADEX^{™}), agarose (SEPHAROSE^{™}). Preferably, the solid support is a sepharose microbead having a diameter between about 2 microns and about 200 microns, or preferably averaging about 30 microns.

In exemplary embodiments, the oligonucleotide-linked enzyme substratesmay be synthesized to contain a single "query position" alongside a unique sequence associated with the substrate (a "Substrate ID") as depicted in **FIG. 2B****.** Query positions may be created in a sequence that forms a hairpin double stranded DNA, such that the query environments all have similar duplex character. A nucleic acid hairpin refers to a region of a single-stranded nucleic acid that contains a duplex (i.e., base-paired) stem and a loop, formed when the nucleic acid comprises two portions that are sufficiently complementary to each other to form a plurality of consecutive base pairs. The hairpin substrates contain 5'-blocking groups and terminal phosphorothioate linkages that inhibit ligation to adaptors and degradation by cellular exonucleases, precluding their recovery by PCR and thus, the generation of false positive DNA repair signal generation.

The hairpin DNA substrates have common sequences at their 3' ends that facilitate base-pairing to a common DNA splint and attachment to beads via splinted DNA ligation. Each oligonucleotide linked to the solid support is typically about 100 bp in length and contains nucleotide sequences that act as identifiers in subsequent analysis steps. The first is an individual bead identification sequence, which identifies the bead which the oligonucleotide is attached to. The second is a unique molecular identifier sequence. The third is a substrate identification sequence which identifies the specific type of DNA adduct that is present on the oligonucleotide. Each of the individual bead identification sequence, the unique molecular identifier sequence, and the substrate identification sequence, are typically between 5 and 25 nucleotides in length, although any length could be used so long as the sequence is unique. The unique molecular identifier sequence may further comprise an endonuclease recognition sequence, which can be utilized in restriction length fragment generation for identification of specific enzymatic activities described below.

As noted above, the DNA oligonucleotide may contain a query position, such as a DNA adduct of various types that act as a substrate for DNA repair enzymes. These query positions may initiate DNA repair activities including those for base excision, mismatch, and nucleotide excision and incision, ribonucleotide excision, topoisomerase-mediated ribonucleotide repair, homologous recombination (HR), non-homologous end joining (NHEJ), translesion DNA synthesis (TLS), and direct reversal. For example, the query position may be a nucleotide base pair mismatch (testing mismatch repair capability/capacity), or nucleotides comprising the wrong base (testing base excision repair) or the wrong sugar (testing nucleotide excision repair), a single nucleotide adduct (for example, O⁶-methylguanine) (testing direct repair), a dinucleotide adduct (such as a platinum adduct formed by a chemotherapeutic drug) (testing nucleotide excision repair), a blunt end double strand break (testing HR), a double strand break with compatible or non-compatible overlapping ends (testing NHEJ), or a cyclobutane pyrimidine dimer (CPD; such as thymine-thymine 6-4 photoproduct) (testing translesion DNA synthesis activity).

The oligonucleotides (which may be linked to a solid support) are incubated with the single cell contents in the small volume reaction chamber (such as a droplet of the emulsion described above) under physiological conditions comprising buffer, pH, and temperature conditions that facilitate the enzymatic activity of enzymes present in the cellular contents of the lysed cell. The incubation typically ranges from 5 to 60 minutes, at a temperature between room temperature and 42 °C, and at a pH between pH 5 and pH 8. The incubation time may be extended to 1-8 hours depending upon the kinetics and efficiency of the repair event that is being tested.

Following the incubation, the oligonucleotides are separated from the reaction. In the case of oligonucleotides linked to a solid support, this separation is facilitated by removing the solid support from the cell lysate, for example by washing the cell lysate from the solid support.
For example, if the solid support, such as a bead, comprises a magnetic material, the bead may be collected on a magnetic surface. The solid support in an emulsion droplet may be recovered by breaking the emulsion and collecting the solid support particle(s) for analysis within microfluidics devices. The solid support in a valve-based microfluidics system may be released from microfluidic channels by opening one or more pressure-controlled valves, directing the solid support to detection/analytical devices within microfluidics devices. The solid support in a nanowell may be analyzed by picking the components out of the well or characterized within each individual well.

The oligonucleotides (including those linked to a solid support particles) may optionally be "polished," for example by strand incision at abasic sites (by the apurinic/apyrimidinic endonuclease APE1) and 5'phosphorylation (by PNKP) to generate 5' ends that are competent for ligation. An adaptor may then be ligated to the 5' end of the oligonucleotide.

The oligonucleotides may be analyzed directly for evidence of enzymatic activity, as described below, or they may be amplified by polymerase chain reaction (PCR) amplification using PCR primers specific to the ligated adaptor and a common sequence at the 3' end of the oligonucleotide. In the PCR, three steps, i.e., a step of thermally denaturing doublestranded DNA into single-stranded DNAs, a step of binding primers to the single-stranded DNAs, and a step of elongating the DNAs with a DNA polymerase are repeated as one PCR cycle to amplify a nucleic acid of interest. The PCR cycle is preferably repeated 40 times to 60 times. This can produce millions of oligonucleotide molecules each having the individual cell identification sequence, the enzyme substrate identification sequence, and the unique molecular identifier sequence. These amplified oligonucleotide molecules are free of any covalent link to the solid support. In instances in which the DNA oligonucleotide query position is a double strand break, the PCR primers will not produce a PCR product during amplification unless a double strand break repair event (i.e., HR or NHEJ) has occurred.

The PCR products may then be analyzed for evidence of enzymatic action on the enzyme substrate. This may include, for example, analysis by restriction fragment length analysis or by direct sequencing. If a restriction fragment length analysis is performed, the PCR products may be incubated with a restriction enzyme that produces two or more identifiable DNA fragments of the PCR product, and these fragments are separated and identified by performing a size-based separation method, such as capillary electrophoresis, or gel electrophoresis, such as agarose or polyacrylamide gel electrophoresis. In embodiments in which the enzyme substrate is a substrate for a DNA repair enzyme, the separated DNA fragments may be indicative of DNA repair activity performed on the oligonucleotides containing a specific substrate identification sequence. In this way, the associated substrate identification sequence serves as a marker of the type of DNA repair activity present in the contents of the lysed cell. In instances in which the substrate identification sequence comprises an endonuclease recognition site and that endonuclease is used to fragment the PCR products, the production of the expected lengths of the PCR fragments serves as a marker of the presence of the substrate identification sequence and therefore the known DNA repair activity within the cell.

In another example, the oligonucleotides may be analyzed for evidence of enzymatic action on the enzyme substrate which is a peptide substrate. For example, in embodiments in which the enzyme substrate is a phosphorylation site in a peptide, the oligonucleotides linked to the enzyme substrate peptide may be analyzed for the presence of phosphorylation, such as by probing the oligonucleotides using an anti-pTyr antibody to detect phosphorylation at a tyrosine within the enzyme substrate peptide.

Alternatively or additionally, the PCR products may be directly sequenced to obtain the full nucleotide sequence of the PCR products, including the sequence of the individual bead identification sequence and the substrate identification sequence. Applying these methods to a population of cells, each analyzed with one or more unique enzyme substrates for DNA repair, the sequencing requirement will be substantial, so the DNA sequencing procedure is preferably high-throughput sequencing (HTP or "second generation" or "next generation") methods. Such sequencing can provide information on the types of enzymatic activity undertaken by the cellular enzymes within the contents of the lysed cell, as well as the types and relative frequencies of the DNA repair types made, thereby providing a broad and robust evaluation of the repair capacity of the tested cell.

Disclosed, but not part of the invention, is a reagent kit comprising components useful in practicing the methods of this disclosure, which kit may include a composition of oligonucleotides, each comprising an enzyme substrate and an individual cell identification sequence, a substrate identification sequence associated with a specific type of enzyme substrate, and a unique molecular identifier sequence. The kit may further include PCR primers, one or more reagents necessary for cell lysis, microemulsion droplet formation, nanowells, and/or other necessary reagents, an instruction booklet, and the like. In some cases, the oligonucleotide-linked enzyme substrate or any kit reagent may be associated with a solid support.

### EXAMPLES

### Example 1

### Oligonucleotide synthesis and bead preparation

Oligonucleotides are synthesized containing a single "query position" alongside a unique sequence associated with the substrate (a "Substrate ID") (**FIG. 2B**). Query positions are created in a sequence that forms a hairpin double stranded DNA, such that each query is formed in a region having similar duplex character. Hairpin DNA substrates have common sequences at their 3' ends that facilitate base-pairing to a common DNA splint and attachment to beads via splinted DNA ligation (**FIG. 2C**) and include restriction enzyme sites within the Substrate ID that are unique for each repair substrate (**FIG. 3**). This approach enables the preparation of mixtures of query substrates on single beads by mixing multiple DNA repair substrates together prior to splint annealing and ligation (**FIG. 2C**). Each bead may have approx. 100 million uniquely addressable substrates, and therefore each query substrate can be represented by thousands of individual hairpin DNA molecules on each bead.

As depicted in **FIG. 5****,** beads were created with immobilized hairpin substrates that contained modifications including:
1) uracil in both A-U and G-U paired contexts;
2) a G ribonucleotide (rG) paired to a C; and
3) a mismatched A-A base-pair.

### Example 2

### Measuring DNA repair activity in extracts

Beads linked to query DNA substrates were incubated with cell-free extracts prepared from:
1) Hap1 and derivative UNG-/- cell lines in which hUNG2 has been ablated by CRISPR/Cas9, eliminating the majority of uracil repair in these cells, and
2) the colon cancer cell line HCT116, which contains a mutation in MLH1 gene and is expected to have defects in mismatch repair.

Extracts were prepared with the commercial MPER cell lysis reagent supplemented with 2.5 mM MgCl₂. Bead immobilized DNA substrates were reacted with extracts for 2 hours. Beads were recovered and sites of repair (e.g., single-stranded gaps or nicks) were captured via second-strand DNA synthesis, adaptor ligation, and PCR (FIG. 3). The method relies on DNA repair enzymes (present in the contents of the cellular extracts) for strand incision at abasic sites (by the apurinic/apyrimidinic endonuclease APE1), and 5' phosphorylation (by polynucleotide kinase phosphatase, PNKP) to generate 5' ends that are competent for ligation. Unreacted (i.e., unrepaired) DNA hairpin substrates contain 5'-blocking groups and terminal phosphorothioate linkages that inhibit ligation to adaptors and degradation by cellular exonucleases, precluding their recovery by PCR.

### Example 3

### Analysis of repair products

The inventors assayed DNA repair activity in two different ways:
First, PCR and restriction analysis was used to measure the specific DNA repair activities present in the cellular extracts (**FIGS. 3** **and** **4A**). As expected, products of uracil base excision were captured by recombinant enzyme and Hap1 extract on the A-U hairpin substrate (**FIG. 4B****,** lanes 4 and 6), whereas we failed to capture uracil repair using UNG-/- extracts (**FIG. 4B**, lane 8). This assay was also used to evaluate whether uracil repair activities could be detected at concentrations relevant to a single-cell droplet-based assay. Previous estimates found that single human B-cells contain approx. 400,000 molecules of UNG2. Because a single cell would be lysed in a 50 pL droplet, the inventor prepared extract from 1 million Hap1 cells in 100 µL of lysis buffer, yielding the same concentration of activities as would be present in a droplet. The PCR- and restriction-based assays for uracil detection were performed on serially-diluted extract and uracil repair was detected in a 100-fold dilution of the starting extract (**FIG. 4C**) suggesting that uracil repair activities can be readily detected in a droplet-based assay.

Second, PCR products depicted in **FIG. 3** were subjected to Illumina high-throughput DNA sequencing, which reveals the site of incision, Substrate ID, UMI and Bead ID in a single long (125 cycle) read. The sequences were aligned to each unique hairpin sequence and the number of 5'-termini (i.e., sites of incision) at each position within the hairpin (**FIG. 5**) were counted.

These data demonstrate that DNA strand incisions are readily detected by the assay with high precision. Expected substrate specificities are recapitulated by the assay (e.g., uracil base excision for the A-U substrate, **FIG. 5A****;** RNase H2 and topoisomerase-mediated ribonucleotide excision, **FIG. 5C**), and unexpected and possibly novel repair modes are readily detected (e.g., combination of uracil base excision and mismatch repair of the G-U substrate, **FIG. 5B**). Finally, there is minimal off-target strand incision: analysis of an unmodified DNA hairpin revealed no specific strand incision (signals were of similar magnitude to those captured in **FIG. 5A****,** positions 1-20, with no specific cleavage in the hairpin substrate). Defects in mismatch repair using the HCT116 cell line were expected (**FIG. 5**, green lines), but there was no observed reduction in mismatch repair using HCT116 extract for either the G-U or A-A hairpins (**FIG. 5B****, D**). DNA mismatch repair protein MLH1 is not required for G-U mismatch repair, possibly explaining why signals from HCT116, which does not express MLH1, are similar to Hap1 for the G-U substrate. The lack of a defect for the A-A substrate in HCT116 extract is not explained, but the assay can nevertheless identify presumptive mismatch repair events using the method (FIG. 5D).

### Example 4

### Single-cell functional assays directly measure enzymatic activities

We developed a method to measure DNA repair activities in single-cell extracts. By including polyadenylated DNA hairpin substrates with defined chemical modifications in a single-cell mRNA-sequencing experiment, we can measure the activity of cognate DNA repair enzymes by analyzing their conversion to repair intermediates and products. The overall repair enzyme activity measurement assay is depicted in **FIG. 6C****.** Products of repair are identified via several molecular steps depicted in **FIG. 7****,** including end repair, A-tailing, and ligation of a double stranded adaptor, followed by PCR. The molecular steps depicted in **FIG. 7** illustrate embodiments of the analytical methods of the present disclosure which are conducted in soluble form (i.e., in the absence of solid support to capture the enzyme substrate-linked oligonucleotides). The method illustrated in **FIG. 7** may be used to analyze DNA repair capacity in a cell that may be present within a mixed population of cells. The DNA repair enzyme substrate is provided as a polyadenylated hairpin DNA containing a lesion that can be repaired by DNA repair enzymes (Ribonucleotide Excision Repair (RER) and/or Base Excision Repair (BER) as illustrated in FIG. 7). Following lesion repair by DNA repair enzymes present in the cell lysate, the remaining polyadenylated and repaired portion of the original polyadenylated hairpin DNA substrate is captured by binding to a dT-tagged oligonucleotide comprising a unique molecular identifier (UMI) and a cell barcode. End repair of the annealed polyadenylated hairpin DNA substrate bound to the dT-tagged oligonucleotide creates blunt ends, which are ligated to a double stranded DNA adaptor that includes a sequence complementary to a PCR primer. PCR amplification of the polyadenylated hairpin DNA substrate creates many copies of a molecule containing 1) the site of strand incision; 2) a unique molecular identifier (UMI) used for counting single repair events; and 3) a cell barcode that is associated with those from the mRNA expression fraction. These molecules are analyzed by DNA sequencing (followed by sequencing (Illumina high-throughput DNA sequencing as illustrated in FIG. 7) to count DNA repair events in each single-cell reaction, providing an assessment of the DNA repair capacity of the cell.

As proof of concept, we performed a single-cell mRNA sequencing experiment with a mixture of two Hap1 cell lines with deletions in either the UNG gene (catalyzes uracil excision) or RNASEH2C (catalyzes ribonucleotide excision). Polyadenylated hairpins containing either a uracil or a ribonucleotide were added to the single cell suspension (**FIGS. 6A** and **6B**) prior to capture. Referring to **FIG. 8A****,** expected sites of repair are shown with the expected sites of incision marked with grey boxes. Cells were scored either UNGKO (**FIG. 8B**) or RNASEH2CKO (**FIG. 8C**) based on whether they exhibited >5% of the maximum counts at the expected sites of repair. Observed sites of hairpin strand incision from aggregated single-cell measurements reflect repair status: UNGKO cells fail to incise uracil-containing hairpins (**FIG. 8B**) and RNASEH2KO cells fail to incise ribonucleotide-containing hairpins (**FIG. 8C**). As shown in FIGS. 8B and 8C, the single cell data largely reflect data from the same assay performed with bulk cell extracts.

Cell mixing was evaluated using a "functional barnyard" approach in which UNGKO and RNASEH2CKO Hap1 cells were mixed. In **FIG. 9** DNA repair activities are plotted at different levels of repair for each detected cell with dots scaled to the number of cells in each bin. Cells were scored either UNGKO or RNASEH2CKO based on whether they exhibited >5% of the maximum counts for either repair activity. Cells along the x- and y-axes represent single UNGKO or RNASEH2CKO cells.

The different ell types were clustered by mRNA expression to identify major classes and plotted as a t-SNE projection. Uracil repair activity (**FIG. 10A**) and ribonucleotide repair activity (**FIG. 10B**) were superimposed and clearly delineate the two major cell types in the experiment. For comparative purposes, levels of UNG and RNASEH2C mRNA are plotted on the bottom panels of **FIGS. 10A** and **10B****,** demonstrating that these mRNA expression data alone are not sufficient to classify cell types.

The various features and processes described above may be used independently of one another, or may be combined in various ways. All possible combinations and subcombinations are intended to fall within the scope of this disclosure. In addition, certain method or process blocks may be omitted in some implementations. The methods and processes described herein are also not limited to any particular sequence, and the blocks or states relating thereto can be performed in other sequences that are appropriate. For example, described blocks or states may be performed in an order other than that specifically disclosed, or multiple blocks or states may be combined in a single block or state. The example blocks or states may be performed in serial, in parallel, or in some other manner. Blocks or states may be added to or removed from the disclosed example embodiments. The example systems and components described herein may be configured differently than described. For example, elements may be added to, removed from, or rearranged compared to the disclosed example embodiments.

Conditional language used herein, such as, among others, "can," "could," "might," "may," "e.g.," and the like, unless specifically stated otherwise, or otherwise understood within the context as used, is generally intended to convey that certain embodiments include, while other embodiments do not include, certain features, elements, and/or steps. Thus, such conditional language is not generally intended to imply that features, elements and/or steps are in any way required for one or more embodiments or that one or more embodiments necessarily include logic for deciding, with or without author input or prompting, whether these features, elements and/or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list.

While certain example embodiments have been described, these embodiments have been presented by way of example only, and are not intended to limit the scope of the invention which is defined by the appended claims. Thus, nothing in the foregoing description is intended to imply that any particular feature, characteristic, step, module, or block is necessary or indispensable.

## Claims

1. A method of analyzing enzymatic activities in a single cell comprising:
a) lysing a single mammalian cell in the presence of one or more oligonucleotides conjugated to enzyme substrates, wherein each oligonucleotide comprises nucleotide sequences comprising:
i. a cell identification sequence associated with a specific cell;
ii. a substrate identification sequence associated with a specific type of enzyme substrate; and,
iii. a unique molecular identifier sequence;
b) incubating the substrate-oligonucleotide conjugates with the lysed single mammalian cell contents under conditions in which enzymes within the cell contents can act specifically on the enzyme substrate;
c) separating reacted and unreacted substrate-oligonucleotide conjugates from the single cell contents;
d) amplifying oligonucleotides associated with reacted substrates by polymerase chain reaction (PCR) to produce amplified DNA products; and,
e) analyzing the amplified DNA products to determine at least one of an amount and a type of enzymatic activity in the cell.

2. The method of claim 1, wherein the single cell is a mammalian cell selected from the group consisting of a cell excised from a living, a cell excised from a biopsy sample, a cell excised from a population of cultured cells, a cell shed from a tissue, a cell isolated from the blood circulation of a mammal, a cell washed and recovered during a surgery, a cell previously treated with a chemotherapeutic agent, a tumor cell, an immunological cell, and a cell that has been obtained or isolated from blood, urine, sweat, sputum, feces, cerebrospinal fluid, ascites, pleural effusion, bile, pancreatic fluid of a mammal.

3. The method of claims 1 or 2, wherein the cell is a human cell.

4. The method of any one of the preceding claims, wherein the cells are lysed by a means selected from freeze-thaw disruption, osmotic disruption, mechanical disruption, ultrasonic disruption, enzymatic disruption, or chemical disruption.

5. The method of any one of the preceding claims, wherein the cells are lysed by chemical disruption following contact with at least one of non-ionic detergents, polyethoxyethanol lauryl ether, polysorbate 20, polyethoxyethanol sorbitan monolaureate, polyethylene lauryl ether, ionic detergents, or combinations thereof.

6. The method of any one of the preceding claims, wherein the single cells are isolated with an oligonucleotide-linked solid support within a microfluidic device in a reaction chamber selected from an emulsion droplet, a nanowell, and between valves in a microfluidic channel.

7. The method of claim 6, wherein the droplets are aqueous droplets formed within a water-in-oil emulsion; optionally wherein the reaction chamber is a nanowell loaded at low densities to achieve a single cell per well, or wherein the reaction chamber is formed between at least two valves formed by elastomeric membranes in a microfluidic channel, or wherein the geometry of the solid support is selected from the group consisting of beads, pellets, disks, and rods.

8. The method of any one of the preceding claims, further comprising reacting oligonucleotides with an abasic endonuclease followed by phosphorylation, and ligating a single strand DNA adaptor to the oligonucleotide, prior to step d) of amplifying the oligonucleotide by polymerase chain reaction.

9. The method of any one of the preceding claims, wherein the DNA adduct comprises a nucleotide aberration that initiates a DNA repair activity selected from base excision, mismatch, nucleotide excision and incision, ribonucleotide excision, topoisomerase-mediated ribonucleotide repair, homologous recombination, non-homologous end joining, translesion DNA synthesis, and direct reversal; optionally wherein the DNA adduct comprises at least one adduct selected from the group consisting of a nucleotide base pair mismatch, a nucleotide comprising the wrong base, a nucleotide comprising the wrong sugar, a single nucleotide adduct, a dinucleotide adduct, a blunt end double strand break, a double strand break with compatible or non-compatible overlapping ends, and a cyclobutane pyrimidine dimer.

10. The method of claim 1, wherein the enzyme substrate comprises a polypeptide comprising a phosphorylation site for a kinase enzyme optionally wherein the phosphorylation site is a tyrosine amino acid residue.

11. The method of claim 1, wherein the enzyme substrate comprises a polypeptide comprising a polypeptide recognition sequence for a protease enzyme; optionally wherein the protease is a caspase enzyme, and the caspase enzyme activity is indicative of programmed cell death activity in the cell.

12. The method of any one of the preceding claims, wherein the cell identification sequence, the unique molecular identifier sequence, and the substrate identification sequence, are each between 5 and 25 nucleotides in length.

13. The method of any one of the preceding claims, wherein the substrate-oligonucleotide conjugate is incubated with the single cell contents in the reaction under physiological conditions comprising buffer, pH, and temperature conditions that facilitate the enzymatic activity present in the cellular contents of the lysed cell.

14. The method of any one of the preceding claims, further comprising capturing polyadenylated mRNA from the cell lysate and creating a cDNA library from the polyadenylated mRNA; optionally wherein the cDNA library is analyzed to obtain gene expression data specific to the single mammalian cell.

15. The method of any one of the preceding claims, wherein analyzing the amplified DNA products comprises high throughput sequencing of at least a portion of the PCR products to identify the sequence of the oligonucleotide, and analysis of the DNA sequence information to identify at least one of the type and the quantity of the enzymatic activities performed by the contents of the lysed cell.

## Patentansprüche

1. Verfahren zum Analysieren von enzymatischen Aktivitäten in einer einzelnen Zelle, umfassend:
a) Lysieren einer einzelnen Säugetierzelle in Anwesenheit von einem oder mehreren Oligonukleotiden, die mit Enzymsubstraten konjugiert sind, wobei jedes Oligonukleotid Nukleotidsequenzen umfasst, umfassend:
i. eine Zellidentifizierungssequenz, die mit einer spezifischen Zelle assoziiert ist;
ii. eine Substratidentifizierungssequenz, die mit einer spezifischen Art von Enzymsubstrat assoziiert ist; und,
iii. eine eindeutige molekulare Identifizierungssequenz;
b) Inkubieren der Substrat-Oligonukleotid-Konjugate mit den lysierten einzelnen Säugetierzellinhalten unter Bedingungen, unter denen Enzyme innerhalb der Zellinhalte spezifisch auf das Enzymsubstrat wirken können;
c) Trennen der reagierten und nicht reagierten Substrat-Oligonukleotid-Konjugate aus den einzelnen Zellinhalten;
d) Amplifizieren von Oligonukleotiden, die mit den reagierten Substraten assoziiert sind, durch Polymerasekettenreaktion (PCR), um amplifizierte DNA-Produkte zu erzeugen; und,
e) Analysieren der amplifizierten DNA-Produkte, um mindestens eine von einer Menge und einer Art enzymatischer Aktivität in der Zelle zu bestimmen.

2. Verfahren nach Anspruch 1, wobei die einzelne Zelle eine Säugetierzelle ist, die ausgewählt ist aus der Gruppe, bestehend aus einer aus einem Lebewesen entnommenen Zelle, einer aus einer Biopsieprobe entnommenen Zelle, einer aus einer Population kultivierter Zellen entnommenen Zelle, einer aus einem Gewebe ausgeschiedenen Zelle, einer aus dem Blutkreislauf eines Säugetiers isolierten Zelle, einer während eines chirurgischen Eingriffs gewaschenen und gewonnenen Zelle, einer zuvor mit einem chemotherapeutischen Mittel behandelten Zelle, einer Tumorzelle, einer immunologischen Zelle und einer Zelle, die aus Blut, Urin, Schweiß, Sputum, Kot, Liquor, Aszites, Pleuraerguss, Galle oder Pankreasflüssigkeit eines Säugetiers erlangt oder isoliert wurde.

3. Verfahren nach Anspruch 1 oder 2, wobei die Zelle eine menschliche Zelle ist.

4. Verfahren nach einem der vorherigen Ansprüche, wobei die Zellen durch eine Einrichtung lysiert werden, die ausgewählt ist aus Gefrier-Auftau-Aufschluss, osmotischem Aufschluss, mechanischem Aufschluss, Ultraschall-Aufschluss, enzymatischem Aufschluss oder chemischem Aufschluss.

5. Verfahren nach einem der vorherigen Ansprüche, wobei die Zellen durch chemischen Aufschluss nach Kontakt mit mindestens einem von nichtionischen Detergenzien, Polyethoxyethanollaurylether, Polysorbat 20, Polyethoxyethanol-Sorbitanmonolaureat, Polyethylenlaurylether, ionischen Detergenzien oder Kombinationen davon lysiert werden.

6. Verfahren nach einem der vorherigen Ansprüche, wobei die einzelnen Zellen mit einem Oligonukleotid-gebundenen festen Träger in einer mikrofluidischen Vorrichtung in einer Reaktionskammer isoliert werden, die ausgewählt ist aus einem Emulsionströpfchen, einem Nanowell und zwischen Ventilen in einem mikrofluidischen Kanal.

7. Verfahren nach Anspruch 6, wobei die Tröpfchen wässrige Tröpfchen sind, die in einer Wasser-in-Öl-Emulsion gebildet sind, optional wobei die Reaktionskammer ein Nanowell ist, das mit geringer Dichte beladen ist, um eine einzelne Zelle pro Well zu erreichen, oder wobei die Reaktionskammer zwischen mindestens zwei Ventilen gebildet ist, die durch elastomere Membranen in einem mikrofluidischen Kanal gebildet sind, oder wobei die Geometrie des festen Trägers ausgewählt ist aus der Gruppe, bestehend aus Perlen, Pellets, Scheiben und Stäben.

8. Verfahren nach einem der vorherigen Ansprüche ferner umfassend ein Reagieren von Oligonukleotiden mit einer abasischen Endonuklease, gefolgt von Phosphorylierung, und Ligieren eines Einzelstrang-DNA-Adaptors an das Oligonukleotid vor Schritt d) eines Amplifizierens des Oligonukleotids durch Polymerasekettenreaktion.

9. Verfahren nach einem der vorherigen Ansprüche, wobei das DNA-Addukt eine Nukleotidaberration umfasst, die eine DNA-Reparaturaktivität auslöst, die ausgewählt ist aus Basen-Exzision, Fehlpaarung, Nukleotid-Exzision und -Inzision, Ribonukleotid-Exzision, Topoisomerase-vermittelter Ribonukleotid-Reparatur, homologer Rekombination, nichthomologer Endverbindung, Translesion-DNA-Synthese und direkter Umkehrung; optional wobei das DNA-Addukt mindestens ein Addukt umfasst, das ausgewählt ist aus der Gruppe, bestehend aus einer Nukleotid-Basenpaar-Fehlpaarung, einem Nukleotid, das die falsche Base umfasst, einem Nukleotid, das den falschen Zucker umfasst, einem Einzelnukleotid-Addukt, einem Dinukleotid-Addukt, einem Doppelstrangbruch mit stumpfem Ende, einem Doppelstrangbruch mit kompatiblen oder nicht kompatiblen überlappenden Enden und einem Cyclobutan-Pyrimidin-Dimer.

10. Verfahren nach Anspruch 1, wobei das Enzymsubstrat ein Polypeptid umfasst, umfassend eine Phosphorylierungsstelle für ein Kinase-Enzym, optional wobei die Phosphorylierungsstelle ein Tyrosin-Aminosäurerest ist.

11. Verfahren nach Anspruch 1, wobei das Enzymsubstrat ein Polypeptid umfasst, umfassend eine Polypeptid-Erkennungssequenz für ein Protease-Enzym; optional wobei die Protease ein Caspase-Enzym ist und die Caspase-Enzymaktivität indikativ für eine programmierte Zelltodaktivität in der Zelle ist.

12. Verfahren nach einem der vorherigen Ansprüche, wobei die Zellidentifizierungssequenz, die eindeutige molekulare Identifizierungssequenz und die Substratidentifizierungssequenz jeweils zwischen 5 und 25 Nukleotiden lang sind.

13. Verfahren nach einem der vorherigen Ansprüche, wobei das Substrat-Oligonukleotid-Konjugat mit dem einzelnen Zellinhalt in der Reaktion unter physiologischen Bedingungen inkubiert wird, die Puffer-, pH- und Temperaturbedingungen umfassen, die die in dem Zellinhalt der lysierten Zelle vorhandene enzymatische Aktivität ermöglichen.

14. Verfahren nach einem der vorherigen Ansprüche, ferner umfassend ein Einfangen von polyadenylierter mRNA aus dem Zelllysat und Erstellen einer cDNA-Bibliothek aus der polyadenylierten mRNA; optional wobei die cDNA-Bibliothek analysiert wird, um Genexpressionsdaten zu erlangen, die für die einzelne Säugetierzelle spezifisch sind.

15. Verfahren nach einem der vorherigen Ansprüche, ein Analysieren der amplifizierten DNA-Produkte umfassend ein Hochdurchsatz-Sequenzieren mindestens eines Abschnitts der PCR-Produkte zum Identifizieren der Sequenz des Oligonukleotids und Analysieren der DNA-Sequenzinformation zum Identifizieren mindestens einer von der Art und der Menge der enzymatischen Aktivitäten, die durch den Inhalt der lysierten Zelle ausgeführt werden.

## Revendications

1. Méthode d'analyse d'activités enzymatiques dans une cellule individuelle comprenant :
a) la lyse d'une cellule mammifère individuelle en présence d'un ou plusieurs oligonucléotides conjugués à des substrats enzymatiques, chaque oligonucléotide comprenant des séquences nucléotidiques comprenant :
i. une séquence d'identification de cellule associée à une cellule spécifique ;
ii. une séquence d'identification de substrat associée à un type spécifique de substrat enzymatique ; et,
iii. une séquence d'identification moléculaire unique ;
b) l'incubation des conjugués substrat-oligonucléotide avec le contenu d'une cellule mammifère individuelle lysée dans des conditions dans lesquelles les enzymes au sein du contenu de cellule peuvent agir spécifiquement sur le substrat enzymatique ;
c) la séparation des conjugués substrat-oligonucléotide ayant réagi et n'ayant pas réagi du contenu d'une cellule individuelle ;
d) l'amplification d'oligonucléotides associés à des substrats ayant réagi par réaction en chaîne par polymérase (PCR) pour produire des produits d'ADN amplifiés ; et,
e) l'analyse des produits d'ADN amplifiés pour déterminer au moins un parmi une quantité et un type d'activité enzymatique dans la cellule.

2. Méthode selon la revendication 1, ladite cellule individuelle étant une cellule mammifère choisie dans le groupe constitué par une cellule excisée d'un être vivant, une cellule excisée d'un échantillon de biopsie, une cellule excisée d'une population de cellules cultivées, une cellule excrétée d'un tissu, une cellule isolée de la circulation sanguine d'un mammifère, une cellule lavée et récupérée lors d'une intervention chirurgicale, une cellule préalablement traitée avec un agent chimiothérapeutique, une cellule tumorale, une cellule immunologique et une cellule qui a été obtenue ou isolée à partir de sang, d'urine, de sueur, de crachats, de matières fécales, de liquide céphalo-rachidien, d'ascite, d'épanchement pleural, de bile, de liquide pancréatique d'un mammifère.

3. Méthode selon la revendication 1 ou 2, ladite cellule étant une cellule humaine.

4. Méthode selon l'une quelconque des revendications précédentes, lesdites cellules étant lysées par un moyen choisi parmi une rupture de congélation-décongélation, une rupture osmotique, une rupture mécanique, une rupture ultrasonique, une rupture enzymatique ou une rupture chimique.

5. Méthode selon l'une quelconque des revendications précédentes, lesdites cellules étant lysées par rupture chimique suite à un contact avec au moins un élément parmi les détergents non ioniques, l'éther laurylique du polyéthoxyéthanol, le polysorbate 20, le monolauréate de polyéthoxyéthanol sorbitane, l'éther laurylique du polyéthylène, les détergents ioniques ou les combinaisons de ceux-ci.

6. Méthode selon l'une quelconque des revendications précédentes, lesdites cellules individuelles étant isolées avec un support solide lié à un oligonucléotide au sein d'un dispositif microfluidique dans une chambre de réaction choisie parmi une gouttelette d'émulsion, un nanopuits et entre des valves dans un canal microfluidique.

7. Méthode selon la revendication 6, lesdites gouttelettes étant des gouttelettes aqueuses formées dans une émulsion eau dans l'huile ; éventuellement ladite chambre de réaction étant un nanopuits chargé à de faibles densités pour obtenir une cellule individuelle par puits, ou ladite chambre de réaction étant formée entre au moins deux valves formées par des membranes élastomères dans un canal microfluidique, ou la géométrie du support solide étant choisie dans le groupe constitué par les billes, les pastilles, les disques et les bâtonnets.

8. Méthode selon l'une quelconque des revendications précédentes, comprenant en outre la réaction d'oligonucléotides avec une endonucléase abasique suivie d'une phosphorylation, et la ligature d'un adaptateur d'ADN simple brin à l'oligonucléotide, avant l'étape d) d'amplification de l'oligonucléotide par réaction en chaîne par polymérase.

9. Méthode selon l'une quelconque des revendications précédentes, l'adduit de l'ADN comprenant une aberration nucléotidique qui initie une activité de réparation de l'ADN choisie parmi l'excision de bases, le mésappariement, l'excision et l'incision de nucléotides, l'excision de ribonucléotides, la réparation de ribonucléotides médiée par la topoisomérase, la recombinaison homologue, la non-jonction d'extrémités homologues, la synthèse d'ADN de translésion et l'inversion directe ; éventuellement ledit adduit de l'ADN comprenant au moins un adduit choisi dans le groupe constitué par un mésappariement de paires de bases nucléotidiques, un nucléotide comprenant la mauvaise base, un nucléotide comprenant le mauvais sucre, un adduit mononucléotidique, un adduit dinucléotidique, une cassure double brin d'extrémités franches, une cassure double brin avec des extrémités chevauchantes compatibles ou non compatibles, et un dimère de cyclobutane pyrimidine.

10. Méthode selon la revendication 1, ledit substrat enzymatique comprend un polypeptide comprenant un site de phosphorylation pour une enzyme kinase, éventuellement ledit site de phosphorylation étant un résidu d'acide aminé tyrosine.

11. Méthode selon la revendication 1, ledit substrat enzymatique comprenant un polypeptide comprenant une séquence de reconnaissance polypeptidique pour une enzyme protéase ; éventuellement ladite protéase étant une enzyme caspase, et l'activité de l'enzyme caspase indiquant une activité de mort cellulaire programmée dans la cellule.

12. Méthode selon l'une quelconque des revendications précédentes, ladite séquence d'identification de cellule, ladite séquence d'identification moléculaire unique et ladite séquence d'identification de substrat étant chacune d'une longueur comprise entre 5 et 25 nucléotides.

13. Méthode selon l'une quelconque des revendications précédentes, ledit conjugué substrat-oligonucléotide étant incubé avec le contenu de cellule individuelle dans la réaction dans des conditions physiologiques comprenant des conditions de tampon, de pH et de température qui facilitent l'activité enzymatique présente dans le contenu cellulaire de la cellule lysée.

14. Méthode selon l'une quelconque des revendications précédentes, comprenant en outre la capture d'ARNm polyadénylé à partir du lysat de cellule et la création d'une banque d'ADNc à partir de l'ARNm polyadénylé ; éventuellement ladite banque d'ADNc étant analysée pour obtenir des données d'expression génique spécifiques à la cellule mammifère individuelle.

15. Méthode selon l'une quelconque des revendications précédentes, ladite analyse des produits d'ADN amplifiés comprenant le séquençage à haut débit d'au moins une partie des produits de PCR pour identifier la séquence de l'oligonucléotide, et l'analyse des informations de séquence d'ADN pour identifier au moins un parmi le type et la quantité des activités enzymatiques exercées par le contenu de la cellule lysée.
